# EUROPEAN PATENT APPLICATION

(11) **EP 2 119 410 A1**
(43) Date of publication of application: **18.11.2009**
(21) Application number: 09002092.6
(22) Date of filing: 16.02.2009
(51) Int. Cl.: A61B 19/00, A61M 1/02, G07C 9/00, G09F 3/00, G06F 19/00, A61J 1/14

(54) **Apparatus for closing bags for hemotransfusion**

(30) Priority: 16.05.2008 IT BO20080296
(71) Applicant: Bernini, Fabrizio, 52021 Bucine (AR) (IT)
(72) Inventor: Bernini, Fabrizio, 52021 Bucine (AR) (IT)
(74) Representative: Lanzoni, Luciano

(57) **Abstract**

Apparatus (1) for closing bags (5) holding material for hemotransfusion (6) and univocally associated with a given patient and having at least one point (7) for access to the stored material, comprising closing means (12) to either allow or prevent, respectively, the insertion and the withdrawal of the bag (5) into and out said seat (3).
The apparatus comprises a control unit (13) for controlling said closing means (12) in response to patient's identifying data associated with said bag and received from an RFID bracelet or wristband.

## Description

The object of the present invention is an apparatus for closing bags holding material for hemotransfusion.

The apparatuses for closing bags holding material for hemotransfusion are usable for locking the bags containing hemocomponents to be transfused to a given patient.

The need to provided high-safety conditions upon a transfusion operation is widely acknowledged.

In particular, it is known the need of making sure that a patient, previously associated with a given bag containing hemotransfusion material, has such content - and not any other unsuited and possibly even incompatible material - actually transfused thereto.

The risk is due in particular to the fact that the association between the bag and the patient - association being carried out by the transfusional service which hand over the same bag to the operators in charge of the transfusion - be not observed by the latter.

In this transfer it is absolutely necessary that the bag in question be safely transfused to the patient of destination through a mechanism for the control of the patient-bag association and to be used at the bed of the same patient upon the transfusion.

In this view, devices for closing bags which contain hemotransfusion material are known to provide for the insertion of the bag inside a storage container.

Once the bag has been inserted in the storage container, a closing device is applied on the same container so as to prevent any accidental removal of the bag therefrom.

The known closing devices also comprise codes applied to the container and/or bag to establish a univocal association with the patient for whom a given bag has been prepared.

Also known are informatics-based systems which collect from the transfusional centre data relative to bags and to patients associated therewith. In these systems, there are predisposed supports (for example labels attached to the bags and/or patient-worn bracelets) which contain bags and patients-identifying data that can be read by a suitable, user-operated reader connected to the closing device, so that only when the reader detects that the bag/patient association is correct, the opening of the device is enabled.

Usable codes may be RFID, bar codes or numerical combinations between patient data (for example, bed number) and the bag.

The closing devices above described exhibit however some drawbacks.

A first drawback is given by the fact that by using the known devices it is likely that a user, also with an awkward movement, will remove the bag containing the hemotransfusion material by simply tampering with the storage container.

Moreover, it is indispensable to use a reading device provided with memory and artificial intelligence, such reader however being of non-practical use, having a significant cost and requiring a specific training for use and maintenance thereof by the operators of the transfusions department who are in charge of the same reader.

The object of the present invention is to overcome the above drawbacks by providing an apparatus for closing bags containing hemotransfusion material which ensures a safe association between the bag of material and the receiver by preventing possible errors.

A further object of the present invention is to provide an apparatus, for closing bags containing hemotransfusion material, which results of prompt and easy use inasmuch as it does not require additional reading/controlling devices and storage containers.

These and other objects, which will appear more clearly by a reading of the detailed description that follows, are achieved, according to the present invention, by means of an apparatus, for closing bags containing hemotransfusion material, having constructional and operational characteristics as disclosed in the attached independent claims, further embodiments of the same invention being set forth in the corresponding dependent claims.

The invention is illustrated herebelow in greater detail with reference to the accompanying drawings which represent an exemplary and non-limiting embodiment thereof. In the drawings:
Fig. 1 shows a closing apparatus, according to the present invention, applied to a bag holding hemotransfusion material;

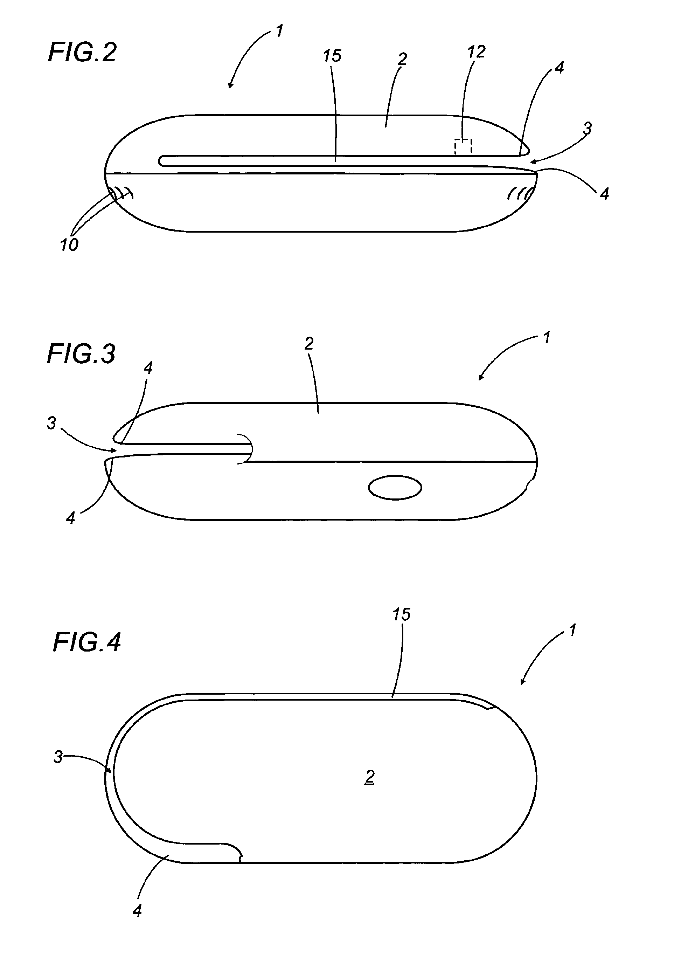

Fig. 2 is a front view of a closing apparatus according to the present invention;
Fig. 3 is a rear view of a closing apparatus according to the present invention;
Fig. 4 is a top view of a closing apparatus shown in the preceding figures;
Fig. 5 is a first side view of a closing apparatus, according to the present invention, applied to a bag for hemotransfusion;

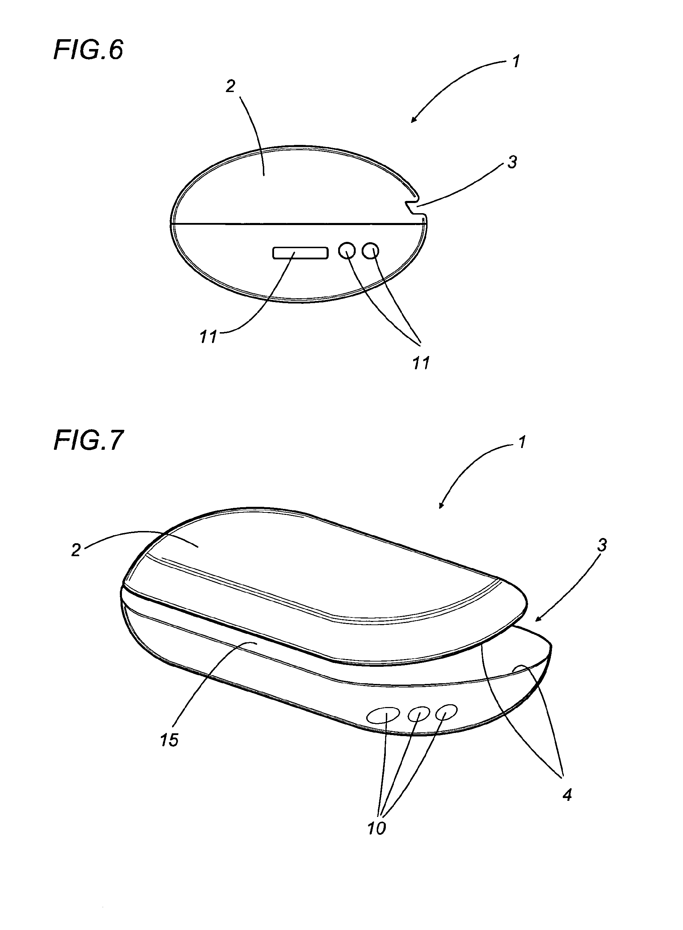

Fig. 6 is a second side view of a closing apparatus, according to the present invention, applied to a bag for hemotransfusion; and
Fig. 7 is a perspective view of the closing apparatus shown in the preceding figures.

Described herebelow with reference to the attached drawings is an apparatus 1 for closing bags 5 holding hemotransfusion material 6 which are univocally associated with a given patient and have at least one point of access (defluxing member) 7 to the material 6 held by said bag.

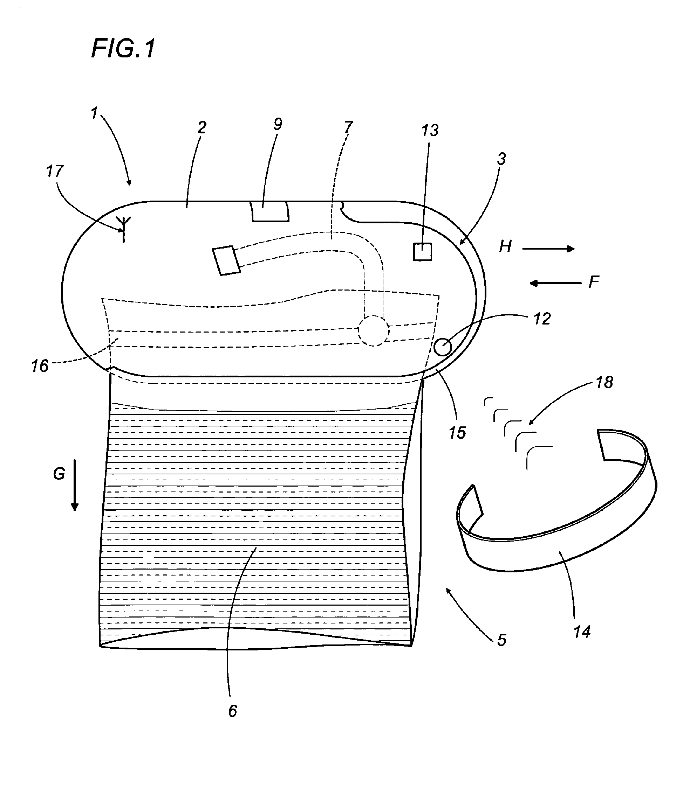

With reference to Fig. 1, a closing apparatus 1, according to the present invention, comprises a body 2, preferably having a shape without cutting edges and portions, which is provided with an internal seat 3 apt to receive the point of access (defluxing member) 7 at a position usually unaccessable from the outside of body 2.

Practically, once the apparatus 1 has been applied to the bag 5 it is possible to reach the content 6 of the bag 5 only by either removing the apparatus 1 or tampering with the same bag 5.

The bag 5 is kept inside the seat 3 by a seat-closing element 12 control-operated between a locking and an unlocking condition.

Advantageously, the closing element 12 is operated by a respective unit 13, solid to the main body 2, by which the unlocking of bag 5 is operated exclusively in response to a data which identifies the patient being associated with the same bag 5.

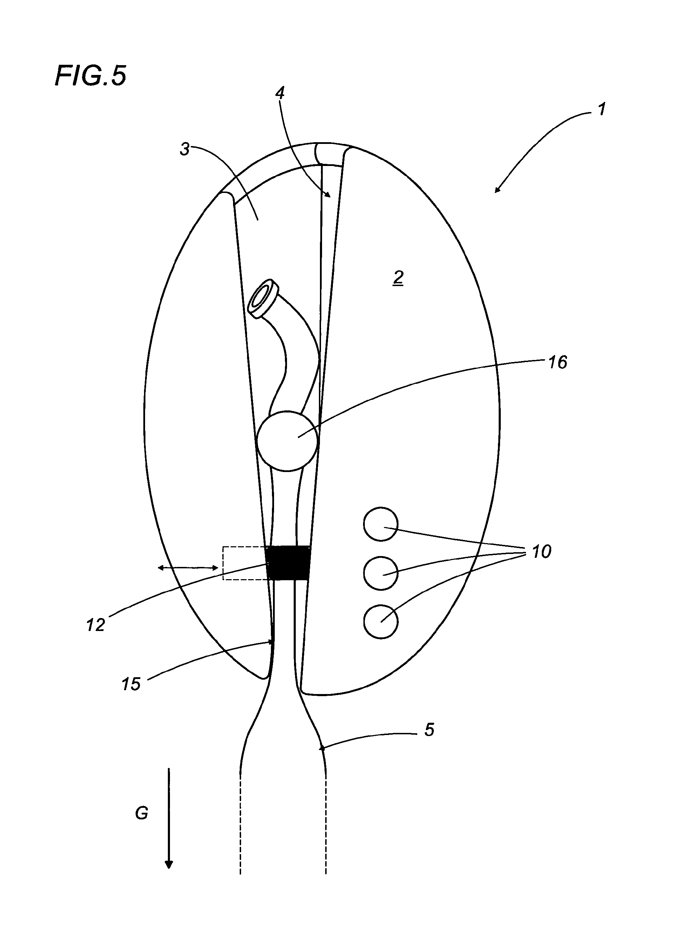

Shown in greater details and with reference to Figs. 1 and 5, is a possible embodiment of a closing apparatus 1, according to the present invention, for application to a bag 5.

The seat 3 of the main body 2 has such a shape as to allow the insertion therein of bag 5 in the direction of arrow "F", while preventing the removal thereof in direction of the arrow "G".

In particular, this characteristic is due to a narrowing of a lower portion 15 of seat 3 located opposite to a an enlarged portion 4, so as to facilitate the introduction of the bag 5 into the same seat 3.

Following the step of inserting the bag 5 inside the seat 3, the closing element 12 is activated by securely blocking the bag against its movement both in direction of arrow "G" and in direction of arrow "H".

In particular, the bag 5 is kept blocked relative to direction "G" inasmuch as the portion 15 has an aperture smaller than the overall dimensions of the components 16 solid to the bag, such as, for example, the points of access (defluxing members) for the exit of transfusional material 6 from the bag 5.

As far as the blockage in the direction of removal (arrow "H" in Fig. 1), this takes place by activating the closing element 12.

Again with reference to Fig. 1, in a preferred embodiment, the patient's identifying data is borne by the bracelets 14 provided with RFDI-type chips, and the control unit 13 for locking/unlocking the element 12 is activated by moving the respective RFID bracelet close to the same apparatus 1.

In this configuration of use, the closing apparatus 1 comprises a reader 17 of RFID type, preferably disposed inside the storage body 2.

The activation of the control unit 13 takes place when the reader 17 reads an identifying code of the bracelet 14 and thus of the patient.

In particular, the closing apparatus 1 rémains in locked configuration, that is, with the element 12 being active, until the reader 17 is at such a distance as to receive the unlocking signal 18 from the respective bracelet 14 associated therewith.

This characteristic allows achieving a high safety in the association of the patient with the respective bag 5.

Advantageously, the apparatus 1 also comprises a unit for memorizing the variations of state of same apparatus 1 and, in particular, the data concerning the opening of apparatus 1 and the monitoring of the operating temperature to which the bag 5 containing the transfusional material 6 has been subjected, the memorization unit making it possible also to retrieve history data concerning the association of the patient with the respective closing apparatus 1.

Advantageously, the closing apparatus 1, according to the present invention, further comprises a safety seal 9 by which it is possible to realize a forced opening, if any, of the same apparatus 1, as necessary upon a failure.

Also advantageously, a closing apparatus 1 according to the present invention comprises one or more light indicators 10, such as of led-type, for signalling the operating condition of the apparatus 1.

In a preferred solution, the led(s) 10 may take up different colourings, each of which being associated with a different operating condition, for example: fixed light to indicate that the recharge of apparatus 1 is under way; yellow light to indicate a stand-by condition; green light to indicate that the apparatus 1 is associated with a respective bag; red light, to indicate a malfunction or problems relative to the operating temperature.

The closing apparatus 1 according to the present invention is able to operate either in a self-contained configuration or being interfaced with one or more software instruments for hospital management.

In the first case, the patient-identifying data are manually put in the apparatus 1 by using a software resident in the same apparatus; whereas in the second case, the association between the apparatus 1, and thus the bag 5, and the respective patient takes place automatically via interface between the same apparatus 1 and the informatics system for hospital management.

At the end of use of the closing apparatus 1, this is taken again to the transfusional centre to unload any data concerning the variations of operational condition, that is, the operations carried out on the same apparatus 1.

Preferably, the transfer of data occurs the moment the apparatus 1 is again connected to the transfusional-management system to be prepared for a fresh use.

Advantageously, the closing apparatus 1 according to the present invention is powered by rechargeable batteries, for example via A USB 11 type transmission, said batteries allowing, indicatively, an autonomy of use of about 100-200 hours.

A possible example of operation of a closing apparatus 1 according the invention is as follows.

Provided within the transfusional centre is a plurality of apparatuses 1 connected to a suitable recharge support, for example, an outlet of multiple USB-type connection.

The moment a request for a transfusion to a given patient arrives at the transfusional centre's operational software, the latter activates a single apparatus 1 - as indicated by the switching-on of a light indicator 10 - applied by a person in charge of the closing of a bag 5 containing the appropriate transfusional material 6.

In particular, the association between users and transfusional material takes place in the following way.

The transfusional centre's operational software, after receiving a request for transfusion to a given patient, associates the latter with a bag 5 holding compatible material.

After having associated the bag 5 with the patient, the software transmits the patient's personal data, and the data relevant to the previously associated bag 5, to a single apparatus 1 located on the suitable support.

The apparatus 1, associated with the given bag 5 by the software, indicates - by the switching-on of a led 10 for example - to the transfusional centre operator, that the association between the same apparatus 1 and the bag 5 and, thus, between the apparatus 1 and the selected patient, has taken place.

At this point, the operator in charge introduces the selected bag 5 into the seat 3 of the respective closing apparatus 1, wherein the locking element 12 becomes active, and hands over the whole to the ward operator who will have to provide for the transfusion.

Upon the transfusion, the operator will have only to move the apparatus 1 close to the bracelet 14 of the associated patient.

If the association is correct, that is, if the operator identifies correctly the patient awaiting for that very bag 5, the closing element 12 is activated as above described to allow the removal of the bag 5 and its use. Otherwise, the bag remains unusable.

The advantages obtained by the apparatus according to the invention consist in a great ease of use of same apparatus 1 and a safe association between patient and bag.

Further advantages of said apparatus lie in the fact that those in charge of the hospital transfusional management system are able to monitor the operational conditions thereof relative also to a high number of patients.

The present industrial invention has been described with reference to a preferred embodiment thereof, but it will be appreciated that equivalent modifications may be made without departing from the scope of protection granted thereto.

## Claims

1. Apparatus (1) for closing bags (5) holding material for hemotransfusion (6) and univocally associated with a given patient and having at least one point (7) for access to the stored material, comprising:
- a main body (2) provided with an internal seat (3) apt to receive the point(s) of access (7) at a position usually unaccessable from the outside of body (2),
- means (12) for closing the seat (3) which are operable under control between a locking and an unlocking condition of the bag (5), to allow or prevent, respectively, the introduction or removal of the access point (7) of bag (5) into and out of said seat (3),
- a control unit (13) solid to said main body (2), for controlling said closing means (12) so as to operate the unlocking of the bag (5) exclusively in response to patient's identifying data associated with said bag (5).

2. Apparatus according to claim 1, wherein said control unit (13) comprises a RFID reader (17) to read RFID codes (18) which identify the respective patient.

3. Apparatus according to any of the preceding claims, further comprising a memorization unit to memorize the variations of state of said apparatus (1).

4. Apparatus according to any of the preceding claims, further comprising one or more light indicators (10) to indicate the operational condition of said apparatus (1).

5. Apparatus according to any of the preceding claims, further comprising an interface (11) to connect said apparatus (1) with one or more informatics system for hospital management.

6. Apparatus according to any of the preceding claims, further comprising a sensor for detecting the temperature of said apparatus.

7. Apparatus according to any of the preceding claims, wherein said seat (3) comprises a first (15) and a second (4) portions for the insertion of said bag (5) into said seat (3), said first portion (15) having an insertion aperture of less dimensions than said second portion (4).

8. Apparatus according to any of the preceding claims, wherein said bag (5) is kept blocked inside the seat (3) with respect to the direction of removal (G), inasmuch as said portion (15) has an aperture less than the overall dimensions of one or more components (16) solid to said bag (5), and with respect to the direction (H) of removal thereof by the actuation of said closing element (12).

9. Apparatus comprising:
a plurality of closing devices (1) according to any of the preceding claims and each comprising at least one interface (11) for receiving data from a hospital management informatics system,
- a first input of data from the management system to receive a request for a bag (5) to be associated with a given patient,
- a processing unit for selecting a bag (5) compatible with said given patient, associating univocally said bag (5) with said patient, and activating a unique apparatus (1),
- means for signalling to an operator which apparatus
(1) is activated.

10. Apparatus according to claim 9 wherein said data-reception interfaces (11) comprise interfaces of USB type.

11. Apparatus according to any of the preceding claims 9 or 10, wherein said signalling means comprise one or more coloured light indicators (10).
